# EUROPEAN PATENT SPECIFICATION

(11) **EP 2 699 218 B1**
(45) Date of publication and mention of the grant of the patent: **17.10.2018**
(21) Application number: 12720634.0
(22) Date of filing: 17.04.2012
(51) Int. Cl.: A61K 8/31, A61K 8/49, A61K 8/67, A61Q 19/02, A61P 17/00, A61K 31/01, A61K 31/353, A61K 31/375, A61K 36/81, A61K 31/352, A61K 36/48, A23L 33/105, A23L 33/15

(54) **USE OF A COMBINATION OF CAROTENOID, PHYTOESTROGEN AND VITAMIN C FOR THE PREVENTION AND/OR TREATMENT OF PIGMENTATION DISORDERS**
VERWENDUNG EINER KOMBINATION EINES CAROTINOIDS, EINES PHYTOESTROGENS UND VITAMIN C ZUR PRÄVENTION UND/ODER ZUR BEHANDLUNG VON PIGMENTSTÖRUNGEN
UTILISATION D'UNE COMBINAISON D'UN CAROTÉNOÏDE, D'UN PHYTOESTROGÈNE ET DE LA VITAMINE C POUR LA PRÉVENTION ET/OU LE TRAITEMENT DE DÉSORDRES PIGMENTAIRES

(30) Priority: 19.04.2011 FR 1153392; 21.06.2011 US 201161499196 P
(43) Date of publication of application: 26.02.2014
(73) Proprietor: NUTRICOS Technologies, 92117 Clichy Cedex (FR)
(72) Inventor: PICCARDI, Nathalie, F-21310 Arceau (FR); BRU, Carole, F-75014 Paris (FR)
(74) Representative: Nony
(86) International application number: PCT/IB2012/051924
(87) International publication number: WO 2012/143856

(56) References cited:
- WO-A1-2009/050101
- WO-A2-02/34233
- DE-A1-102008 009 799
- FR-A1- 2 859 908
- DRENO B: "NOUVELLES METHODS D'EVALUATION APPLIQUEES A UNE ASSOCIATION BREVETEE DE LACTO-LYCOPENE D'ISOLAVONES DE SOJA ET DE VITAMINE C DANS LA CORRECTION DU VIEILLISSEMENT CUTANE//NEW METHODS OF EVALUATION APPLIED TO A PATENTED COMBINATION OF LACTO-LYCOPENE(TM), SOYBEAN ISOFLAVONE AND VITAMIN C IN THE CORRECT", NOUVELLES DERMATOLOGIQUES, STRASBOURG, FR, vol. 22, no. 8, 1 January 2003 (2003-01-01), pages 557-561, XP009068873, ISSN: 0752-5370
- LANGE SKOVGAARD G R ET AL: "Effect of a novel dietary supplement on skin aging in post-menopausal women", EUROPEAN JOURNAL OF CLINICAL NUTRITION, XX, XX, vol. 60, 1 January 2006 (2006-01-01), pages 1201-1206, XP008080469,
- BOLANCA I. ET AL.: "Chloasma - the mask of pregnancy", COLL ANTROPOL, vol. 32 Suppl. 2, 1 October 2008 (2008-10-01), pages 139-141,

## Description

The present invention relates to the field of preventing or treating skin pigmentation disorders, including hyperpigmentation disorders, and focuses on the treatment of melasma and chloasma.

Aesthetic reasons are the main reason for consultation by patients suffering from a skin pigmentation disorder, including a skin hyperpigmentation. In general, skin pigmentation disorders, including skin hyperpigmentations, are asymptomatic and do not entail any medical consequence.

Skin pigmentation disorders occur frequently and are manifested in a variety of different forms. Hyperpigmentation generally results from an increase in melanin, which may arise in the epidermis, the dermis or both. Typically, this takes place via an increase in melanin production by the existing melanocytes or by proliferation of the active melanocytes.

Several factors may be involved in the physiopathology of hyperpigmentation, including: (i) exposure to ultraviolet rays from the sun or other sources (the predominant factor), (ii) genetic factors, (iii) pregnancy, (iv) the use of contraceptives, (v) hormonal treatments (oestrogen replacement therapy and oestrogen treatment for prostate cancer), (vi) the use of photosensitizing and anticonvulsant medicaments, (vii) ovarian or thyroid dysfunction, (viii) the use of certain cosmetic products, (ix) the consequences of a treatment of inflammatory skin eruptions (acne, atopic dermatitis, etc.), and (x) inflammation following dermatological treatments (peeling, dermabrasion, etc.).

Usually, the treatments recommended for hyperpigmentation disorders are depigmenting topical products, chemical peeling treatments, cryosurgery and laser treatment.

By definition, these treatments act locally on the areas to be treated - areas on which they may be unequally distributed, in the case of topical products - and necessitate careful and repeated applications. In certain cases, they may be the cause of secondary skin reactions, or even of discomfort.

There is thus a need for novel active agents in the field of preventing or treating skin pigmentation disorders, which are alternative or improved relative to the known active agents.

Document Dreno B: «Nouvelles méthodes d'evaluation appliquées a une association brevetée de lacto-lycopène d'isoflavones de soja et de vitamine C dans la correction du vieillissement cutané », nouvelles dermatologiques, Strasbourg, FR, vol. 22, no.8, 1 janvier 2003 (2003-01-01), pages 557-561 relates to the assessment of skin aging in patients receiving a dietary supplement comprising lycopene, isoflavones and vitamin C.

Unexpectedly, it is shown according to the invention that a combination of at least one carotenoid, of at least one phytooestrogen and of vitamin C, in particular in specific respective amounts of each of the three constituents, constitutes an active agent for preventing or treating skin pigmentation disorders being melasma or chloasma, when the said combination is administered orally.

The present invention relates to a combination (i) consisting of one carotenoid, preferably lycopene, (ii) one phytooestrogen, preferably an isoflavonoid, and (iii) of vitamin C in a cosmetic composition, for use in the prevention and/or treatment of skin pigmentation disorders, said cosmetic composition being suitable for orally administering a daily dose of from 1 to 25 mg of the said carotenoid, from 10 to 300 mg of the said phytooestrogen and from 10 to 1000 mg of vitamin C, the skin pigmentation disorder being melasma or chloasma.

The possible others components used are auxiliary agents without active effect on the indications considered in the invention.

According to the invention, the term "carotenoid" means either a carotenoid with provitamin A activity or a carotenoid with no provitamin A activity.

Needless to say, according to the invention, the carotenoid may be a mixture of carotenoids with provitamin A activity and of carotenoids with no provitamin A activity. This mixture may be in any proportion.

According to the invention, the carotenoid with provitamin A activity may be a mixture of carotenoids with provitamin A activity. This mixture may be in any proportion.

Among the carotenoids with provitamin A activity, examples that may be mentioned include β-carotene and α-carotene.

According to the invention, the carotenoid with no provitamin A activity may be a mixture of carotenoids with no provitamin A activity. This mixture may be in any proportion. Among the carotenoids with no provitamin A activity, examples that may be mentioned include zeaxanthin, cryptoxanthin, lutein and lycopene.

The carotenoid used according to the invention may be of natural or synthetic origin. The term "natural origin" refers to a carotenoid, in pure form or in solution irrespective of its concentration in the said solution, obtained from a natural element. According to a preferential mode of the invention, a lycopene-rich extract is used, for instance a tomato extract.

The term "synthetic origin" refers to a carotenoid, in pure form or in solution irrespective of its concentration in the said solution, obtained via chemical synthesis.

When the carotenoid is of natural origin, it may be obtained from a plant material derived from the whole plant cultured *in vivo* or derived from *in vitro* cultivation.

The term *"in vivo* cultivation" means any cultivation of standard type, i.e. in soil in the open air or in a greenhouse, or alternatively out of the soil.

The term *"in vitro* cultivation" means all the techniques known to those skilled in the art for artificially obtaining a plant or a plant part. The selection pressure imposed by the physicochemical conditions during the growth of plant cells *in vitro* makes it possible to obtain a standardized plant material that is available throughout the year, in contrast with plants cultivated *in vivo.*

Preferentially, according to the invention, a plant derived from *in vivo* cultivation is used.

Any extraction method known to those skilled in the art may be used to prepare the carotenoid used according to the invention.

The carotenoid may be in alcoholic solution, especially ethanolic solution. The carotenoid may also be in lipidic (oil) or lipoalcoholic solution.

The carotenoids that are preferred according to the invention are β-carotene and lycopene.

Lycopene is most preferentially used.

Needless to say, if a person skilled in the art uses the carotenoid in the form of a solution or a plant extract, for example, he knows how to adjust the solution used in his composition in order for the final amount of carotenoid in the composition to be in accordance with the predefined usable amounts.

Lycopene is a natural pigment found in ripe fruit, particularly in tomatoes. It belongs to the carotenoid family and its structure is similar to that of β-carotene. Lycopene is used in compositions with tanning activity for its role in melanin synthesis (WO 97/47278), in compositions for treating the hair and/or acne for its activity on 5a-reductases (JP-2940964) or as a free-radical scavenger (JP-A-8-283136). Lycopene may be in cis or trans chemical form, and may be of natural or synthetic origin. It is also possible according to the invention to use any process for manufacturing and/or formulating and/or encapsulating lycopene. Thus, an example of a more complex ingredient that is intended is a primary composition comprising lycopene and a whey protein. This primary composition is especially described in document WO 01/91588. This primary composition is also known as lactolycopene.

Vitamin C is commonly used in food supplements for its antioxidant activities, and is recognized by the EFSA (European Food Safety Authority) as contributing towards protecting cells from oxidative damage and contributing towards normal collagen formation and normal skin formation (EFSA Journal 2009: 7(9): 1226 [28 pp.]). Vitamin C may be in the form of ascorbic acid and derivatives thereof (esters, salts, etc.).

Phytooestrogens are a family of non-stearoyl compounds naturally produced by plants, which includes isoflavones, and also the phytooestrogens contained in hop, or lignans, in particular those of flax and of *Schizandra chinensis.*

Isoflavones are natural substances (heterocyclic phenols) belonging to the flavonoid family. In soybean grains, the concentrations of isoflavones (daidzein and genistein) or glycosylated forms thereof (daidzine, genistine) are large. By virtue of their structural similarity to female hormones of oestrogen type, genistein and daidzein are also known as phytooestrogens or phytohormones. In contrast with flavonoids, they are present in only a limited number of plants: soybean (milk, grain, meal) is the main source. As a result of these characteristics, soybean isoflavones are used as food supplements mainly for preventing or slowing down the symptoms of the menopause (Messina, 2010). Isoflavones have also been described for their antioxidant and depigmenting properties (DE-44 32 947).

The prior art discloses the use of a combination of at least one carotenoid with provitamin A activity and at least one carotenoid with no provitamin A activity for treating the signs of ageing (WO 02/34232). The use of a combination of at least one carotenoid and at least one isoflavonoid for treating the signs of ageing of the skin is also known (WO 02/34233).

However, to the Applicant's knowledge, the use of a combination of at least one carotenoid, of at least one phytooestrogen and of vitamin C for preventing and/or treating skin pigmentation disorders being melasma or chloasma has never been described in the prior art.

Preferentially, the said carotenoid is lycopene.

Preferentially, the said at least one phytooestrogen is an isoflavonoid.

For the purposes of the present invention, the term "preventing" means significantly reducing the risk of manifestation of the problem under consideration.

The oral administration of active agents according to the invention has the advantage of acting globally on the skin as a whole, via a rapid mode of administration that imposes few constraints, when compared with the topical administration of active agents.

Furthermore, without wishing to be bound by any theory, the Applicant thinks that, by definition, the oral administration of active agents enables these active agents to reach physiological targets that cannot be accessed by topically administered active agents. In other words, a given combination of active agents is capable of having different activities, depending on whether this combination is administered locally (for example topically) or systemically (for example orally).

It has been shown according to the invention that the combination (i) of at least one carotenoid, preferably lycopene, (ii) of at least one phytooestrogen, preferably an isoflavonoid, and (iii) of vitamin C above, when administered orally at the specified daily doses, makes it possible especially to prevent and treat a skin pigmentation disorder being melasma or chloasma.

The examples below illustrate the results of clinical tests performed on a group of individuals suffering from chloasma, which show that the combination (i) of at least one carotenoid, preferably lycopene, (ii) of at least one phytooestrogen, preferably an isoflavonoid, and (iii) of vitamin C above, when it is administered orally at the daily doses described above, allows the treatment of a skin pigmentation disorder being melasma or chloasma. It is observed that the use of the said combination in the manner in accordance with the invention induces both (i) decolorization of the skin regions affected with hyperpigmentation and (ii) a reduction of the surface area of the skin regions affected with hyperpigmentation.

The examples also show that the combination (i) of at least one carotenoid, preferably a lycopene, (ii) of at least one phytooestrogen, preferably an isoflavonoid, and (iii) of vitamin C above, when administered orally at the daily doses described above, allows the prevention of a skin pigmentation disorder being melasma or chloasma. It is observed that the use of the said combination in the manner in accordance with the invention makes it possible to prevent, in the case of individuals with a predisposition towards developing skin hyperpigmentation disorders, the occurrence of these disorders. It has especially been shown that a use of the said combination in the manner in accordance with the invention, in the case of individuals who have already developed chloasma, makes it possible to block the occurrence of additional regions of hyperpigmented skin.

For the purposes of the invention, the term "skin pigmentation disorder" means any occurrence of a change of colour, or of complexion, of all or part of the surface of the skin, which includes a global or local change of the skin complexion, and also hyperpigmentation disorders.

For the purposes of the invention, the term "skin hyperpigmentation disorder" means any occurrence of excess pigmentation on unaffected skin region, by comparison with the average level of pigmentation of the skin surface of the individual.

The level of pigmentation of a skin surface may be measured using any suitable type of device for analysing colour, for example with a suitable spectrocolorimetry machine, or alternatively a reflectometer machine, which are well known in the state of the art.

Preferably, the level of pigmentation of a skin surface is measured using a suitable standard colour chart. To measure the level of pigmentation, the standard colour chart is applied to the skin so that at least one edge of the chart is adjacent to the skin region to be measured. By visual comparison, the colour degree value on the chart that is the closest to the colour of the skin region being analysed is read.

Furthermore, it has been shown according to the invention that the use of the combination (i) of a carotenoid, preferably lycopene, (ii) of at least one phytooestrogen, preferably at least one isoflavonoid, and (iii) of vitamin C of the invention, at the daily doses specified in the present description, does not simultaneously induce any adverse effects, as is illustrated especially by the absence of effects on the various physiological functions tested, especially by means of urine analyses, faecal analyses and biochemical blood analyses. The results of the examples show especially that the oral use of the said combination, at the specified daily doses, does not induce any change in the renal and hepatic functions.

In other words, it has been shown that the use according to the invention makes it possible to prevent and/or treat skin pigmentation disorders being melasma and chloasma, and is totally harmless to the user.

In particular, the use of a combination (i) of at least one carotenoid, preferably a lycopene, (ii) of at least one phytooestrogen, preferably an isoflavonoid, and (iii) of vitamin Cis described as making it possible to prevent and/or treat any type of non-pathological skin pigmentation disorder, and in particular any type of non-pathological skin hyperpigmentation.

### Non-pathological skin pigmentation disorder

As has already been stated previously, skin pigmentation disorders, and in particular skin hyperpigmentation disorders, are common and are manifested in a variety of different forms. However, the skin pigmentation disorders, in particular the skin hyperpigmentation disorders, with which the invention is concerned may all be defined as involving the occurrence of at least one skin region having a darker colour than the average colour of the skin surface of the individual examined.

Skin pigmentation disorders are materialised by the presence of skin marks of more or less broad area, which are darker in colour than the normal colour of the individual's skin that surrounds the said skin marks.

Skin pigmentation disorders especially include melasma and lentigo.

Melasma (also known as chloasma) is most frequently encountered in the case of pregnant women and women taking anti-ovulation medicaments. Melasma is also known as the "pregnancy mask".

Melasma appears as a large dark, reticulated macule with irregular edges and is mainly located on the cheeks, the upper lip and the forehead.

Melasma is also often encountered in the case of men or women with no detectable endocrine imbalance, but exposure to sunlight is necessary for its development.

Lentigo presents in the form of hyperpigmented skin marks, which may appear at any age, and which are usually darker than the ephelides. Lentigo is especially characterized by an increase in the number of melanocytes in the basal layer. Lentigos especially include (i) solar lentigines, which appear in the cases of individuals with clear skin, on skin regions exposed to sunlight for a long time, (ii) lentiginous pigmentations following skin treatments using UVA (320-400 nm - also known as PUVA-therapy), (iii) multiple lentigines, especially on the palms, the soles of the feet, mucous membranes, or on an exposed skin, or alternatively (iv) lentigines affecting the lips, the vulva or the penis.

Skin pigmentation disorders also include hyperpigmentation situations following inflammation. Post-inflammatory hyperpigmentation is rather independent of the degree of inflammation and depends more on the nature of the trauma that caused the inflammation. Post-inflammatory hyperpigmentations may be superior after certain lesions, such as heat burns. Post-inflammatory hyperpigmentation may persist for months, or even years.

Hyperpigmentation disorders may be chosen from melasma, chloasma, lentigines, senile lentigo, vitiligo, post-inflammatory hyperpigmentations due to abrasion and/or a burn and/or a scar and/or a dermatosis and/or a contact allergy; hyperpigmentations of genetic determinism, hyperpigmentations of metabolic or medicational origin, or any other hyperpigmentary lesions.

In certain embodiments, the use of a combination (i) of at least one carotenoid, preferably a lycopene, (ii) of at least one phytooestrogen, preferably an isoflavonoid, and (iii) of vitamin C in accordance with the invention, is intended for treating and/or preventing melasma or chloasma caused by hormonal changes, in particular in oestrogens or progestogens.

The said hormonal changes may be caused by the administration of a contraceptive composition.

The said hormonal changes may also be caused by the administration of a hormone replacement composition, for example for a treatment of menopause.

In certain embodiments, the use of a combination (i) of at least one carotenoid, preferably a lycopene, (ii) of at least one phytooestrogen, preferably an isoflavonoid, and (iii) of vitamin C in accordance with the invention, is intended for treating and/or preventing the pregnancy mask.

In certain embodiments, the use of a combination (i) of at least one carotenoid, preferably a lycopene, (ii) of at least one phytooestrogen, preferably an isoflavonoid, and (iii) of vitamin C in accordance with the invention, is intended for treating and/or preventing melasma or chloasma caused by exposure to UV rays.

In certain embodiments, the use of a combination (i) of at least one carotenoid, preferably a lycopene, (ii) of at least one phytooestrogen, preferably an isoflavonoid, and (iii) of vitamin C in accordance with the invention, is intended for treating and/or preventing melasma or chloasma on photosensitive skin, photoallergic skin or on skin that is subject to a phototoxicity reaction.

Skin pigmentation disorders may comprise disorders associated with an impairment of the skin complexion.

The use of a combination (i) of at least one carotenoid, preferably a lycopene, (ii) of at least one phytooestrogen, preferably an isoflavonoid, and (iii) of vitamin C is described for treating and/or preventing heterogeneity of the skin complexion.

The use of a combination (i) of at least one carotenoid, preferably a lycopene, (ii) of at least one phytooestrogen, preferably an isoflavonoid, and (iii) of vitamin C is described for improving the skin complexion.

The use of a combination (i) of at least one carotenoid, preferably a lycopene, (ii) of at least one phytooestrogen, preferably an isoflavonoid, and (iii) of vitamin C is described as making it possible to promote and maintain the radiance of the skin complexion, and in particular a homogeneous complexion.

The use of a combination (i) of at least one carotenoid, preferably a lycopene, (ii) of at least one phytooestrogen, preferably an isoflavonoid, and (iii) of vitamin C is described as making it possible to treat and/or prevent an impairment of radiance of the complexion, or a loss of radiance of the skin complexion. The use of a combination (i) of at least one carotenoid, preferably a lycopene, (ii) of at least one phytooestrogen, preferably an isoflavonoid, and (iii) of vitamin C is described as making it possible to give the skin a uniform, luminous, brighter, or even more radiant complexion, which is a reflection of healthy skin.

The use of a combination (i) of at least one carotenoid, preferably a lycopene, (ii) of at least one phytooestrogen, preferably an isoflavonoid, and (iii) of vitamin C is described asmaking it possible to prevent and/or treat an off-colour, dull and/or nonuniform skin complexion, or to prevent and/or treat skin imperfections, chosen in particular from spots, dartres, dyschromia, senescence marks, blackheads and/or melasma, or alternatively to prevent and/or treat a pasty, jaundiced or even sickly complexion.

### Lycopene

Lycopene is a natural pigment found in ripe fruit, particularly in tomatoes. It belongs to the carotenoid family and its structure is similar to that of β-carotene.

Lycopene is a carotenoid with no provitamin A activity.

The role of lycopene in the ripening of fruit is known in the prior art.

Lycopene may be in *cis* or *trans* chemical form.

### Isoflavonoids

Isoflavonoids constitute a subclass of flavonoids, formed from a 3-phenylchroman backbone which may comprise varied substituents and different oxidation levels. In contrast with flavonoids, they are present in only a very limited number of plants.

Isoflavonoids include several classes of compounds, among which mention may be made of isoflavones, isoflavanones, rotenoids, pterocarpans, isoflavanes, isoflavan-3-enes, 3-arylcoumarins, 3-aryl-4-hydroxycoumarins, coumestanes, coumaronochromones, α-methyldeoxybenzoins or 2-arylbenzofurans.

For a complete review of isoflavonoids, their analysis methods and their sources, a person skilled in the art may advantageously refer to chapter 5 "Isoflavonoids" written by P.M. Dewick in The Flavonoids, published by Harbone, pp. 125-157 (1988).

The isoflavonoids that are suitable for use in the present invention may be of natural or synthetic origin. The term "natural origin" means an isoflavonoid in pure form or in solution at various concentrations, obtained via various extraction processes from an element, generally a plant, of natural origin. The term "synthetic origin" means an isoflavonoid in pure form or in solution at various concentrations, obtained via chemical synthesis.

It is preferred to use isoflavonoids of natural origin. Among these, mention may be made of: daidzine, genistine, equol, daidzein, formononetine, cuneatine, genistein, isoprunetine and prunetine, cajanine, orobol, pratensein, santal, junipegenin A, glycitein, afrormosine, retusine, tectorigenin, irisolidone, jamaicine, and also analogues and/or metabolites thereof.

According to the present invention, among the isoflavonoids, it is preferred to use isoflavones, including the aglycone forms and the glycosylated forms of isoflavones. Among the isoflavones, the simplest isoflavones are preferred, among which are daidzein, daidzine, genistein and genistine, equol and mixtures thereof. Daidzein, daidzine, genistein and genistine are in particular present in extract of soybean (*Glycina max*) available from Archer Daniels Midland Company under the name Novasoy®.

Processes for preparing isoflavones are especially described in WO 95/10530, WO 95/10512, US-5 679 806, US-5 554 519, EP-812 837 and WO 97/26269.

In one combination of active agents according to the invention, the isoflavonoids are preferentially chosen from isoflavones, isoflavanones, rotenoids, pterocarpans, isoflavanes, isoflavan-3-enes, 3-arylcoumarins, 3-aryl-4-hydroxycoumarins, coumestanes, coumaronochromones, α-methyldeoxybenzoins and 2-arylbenzofurans.

In particular, the isoflavonoids may be chosen from daidzein, formononetine, cuneatine, genistein, isoprunetine and prunetine, cajanine, orobol, pratensein, santal, junipegenin A, glycitein, afrormosine, retusine, tectorigenin, irisolidone, jamaicine, and also analogues and/or metabolites thereof.

In addition, an isoflavonoid that is suitable for use in the invention may be chosen from genistine, daidzine, genistein, daidzein, glycitine, equol, formononetine, cuneatine, isoprunetine and prunetine, cajanine, orobol, pratensein, santal, junipegenin A, glycitein, afrormosine, retusine, tectorigenin, irisolidone, jamaicine, and also analogues and/or metabolites thereof.

In certain embodiments, the said isoflavonoid is an isoflavone, or a mixture of isoflavones.

In certain embodiments, the said isoflavonoid is chosen from daidzine, daidzein, genistine and genistein, or a mixture thereof.

In certain embodiments, the said at least one isoflavone is in the form of a mixture of isoflavones.

In certain embodiments, the said at least one isoflavone is in the form of a mixture of an extract of soybean isoflavones.

By way of illustration, an extract of soybean isoflavones may be, for example, the extract of soybean (*Glycina max*) available from Archer Daniels Midland Company under the name Novasoy®.

### Cosmetic use

The compositions that are intended for use according to the invention may be in any galenical form normally available for oral administration.

As has already been specified previously, the combination (i) of at least one carotenoid, preferably a lycopene, (ii) of at least one phytooestrogen, preferably an isoflavonoid, and (iii) of vitamin C, which is used orally according to the invention is included in a cosmetic composition that is suitable for administering a daily dose of from 1 to 25 mg of the said carotenoid, preferably of the said lycopene, from 10 to 300 mg of the said phytooestrogen, preferably of the said isoflavonoid, and from 10 to 1000 mg of vitamin C.

In certain embodiments, the said cosmetic composition is suitable for administering a daily dose of carotenoid, in particular of lycopene, ranging from 2 to 12 mg.

In certain embodiments, the said cosmetic composition is suitable for administering a daily dose of carotenoid, in particular of lycopene, ranging from 4 to 7 mg.

In certain embodiments, the said cosmetic composition is suitable for administering a daily dose of vitamin C ranging from 10 to 800 mg, especially from 10 to 600 mg, or even from 10 to 400 mg, and better still from 10 to 250 mg.

In certain embodiments, the said cosmetic composition is suitable for administering a daily dose of vitamin C ranging from 20 to 120 mg.

In certain embodiments, the said cosmetic composition is suitable for administering a daily dose of vitamin C ranging from 50 to 70 mg.

In certain embodiments, the said cosmetic composition is suitable for administering a daily dose of isoflavone(s) ranging from 16 to 100 mg.

In certain embodiments, the said cosmetic composition is suitable for administering a daily dose of isoflavone(s) ranging from 40 to 60 mg.

When a mixture of isoflavones is used in the form of a soybean extract, a daily dose of total isoflavones ranging from 16 to 100 mg, i.e. a daily dose of aglycone isoflavone equivalent (mainly daidzein and genistein) ranging from 8 to 50 mg, may be used.

When a mixture of isoflavones is used in the form of a soybean extract, a daily dose of total isoflavones ranging from 40 to 60 mg, i.e. a daily dose of aglycone isoflavone equivalent (mainly daidzein and genistein) ranging from 20 to 30 mg, may be used.

In particular embodiments, the said cosmetic composition is suitable for administering a daily dose of 6 mg of lycopene, 50 mg of soybean isoflavone and 60 mg of vitamin C.

Needless to say, the oral use of the combination (i) of at least one carotenoid, preferably lycopene, (ii) of at least one phytooestrogen, preferably an isoflavonoid, and (iii) of vitamin C, at the specified daily doses, may be performed using suitable oral compositions, which may also contain several additional active agents, (i) other than the carotenoid, in particular lycopene, (ii) other than the phytooestrogens, in particular isoflavonoids, and (iii) other than vitamin C.

As active agents that may be used, mention may be made of vitamins B3, B5, B6, B8, E, D and PP, curcuminoids, sugars, amino acids, sulfureous amino acids, polyunsaturated fatty acids, probiotics, phytosterols, polyphenol extracts, and minerals such as zinc, calcium or magnesium.

In particular, use may be made of an antioxidant complex comprising selenium and vitamin E, flavonoids such as catechins, hesperidin, proanthocyanidins and anthocyanins, ubiquinones, resveratrol, coffee extracts, probiotics, prebiotics, taurine, amino acids, glutathione precursors and 3- and 6-polyunsaturated fatty acids.

From his general knowledge in the field of the galenical formulation of oral cosmetic compositions, a person skilled in the art can easily prepare cosmetic compositions that are suitable for the use of the invention, and in particular the dosage units of the said cosmetic compositions that are suitable for a daily administration of the combination of active agents at the specified doses.

In certain embodiments of the cosmetic composition, the daily dose of the combination (i) of at least one carotenoid, preferably a lycopene, (ii) of at least one phytooestrogen, preferably an isoflavonoid, and (iii) of vitamin C, is included in a single dosage unit.

In other embodiments of the cosmetic composition, the daily dose of the combination (i) of at least one carotenoid, preferably a lycopene, (ii) of at least one phytooestrogen, preferably an isoflavonoid, and (iii) of vitamin C, is included in a plurality of dosage units.

Thus, the daily dose of the combination of active agents may be contained in 2, 3, 4, 5, 6, 7, 8, 9 or 10 dosage units.

The process according to the invention may comprise a single administration.

According to another embodiment, the administration is repeated, for example 2 to 4 times daily for one day or more and generally for an extended period of at least 4 weeks, or even 4 to 15 weeks with, where appropriate, one or more periods of stoppage.

In one particular embodiment, the daily dose of the combination of active agents is included in two dosage units of the oral cosmetic composition.

For ingestion, numerous embodiments of oral compositions are possible.

Milk, yoghurt, cheese, fermented milks, milk-based fermented products, ice creams, products based on fermented cereals, milk-based powders, baby and infant formulations, food products of candy type, chocolate, cereals, animal feed and in particular pet food, tablets, gel capsules or soft capsules, oral supplements in dry form and oral supplements in liquid form are especially suitable for use as pharmaceutical or food supports.

In certain embodiments, the use according to the invention is characterized in that a cosmetic composition is prepared in which the phytooestrogens, preferably the isoflavone compounds, are present in a content ranging from 0.0001% to 50% by weight, preferentially from 0.001% to 30% by weight and preferably from 0.1% to 10% by weight relative to the total weight of the composition.

In certain embodiments, the use according to the invention is characterized in that a cosmetic composition is prepared in which the carotenoid, preferably lycopene, is present in a content ranging from 0.0001% to 50% by weight, preferentially from 0.001% to 10% by weight and even more preferably in a content ranging from 0.02% to 2% by weight relative to the total weight of the composition.

In certain embodiments, the use according to the invention is characterized in that a cosmetic composition is prepared in which the vitamin C is present in a content ranging from 0.0001% to 50% by weight, preferably from 0.1% to 30% by weight and even more preferably in a content ranging from 0.2% to 12% by weight relative to the total weight of the composition.

In certain embodiments, the use according to the invention is characterized in that a cosmetic composition is prepared in which the phytooestrogens, preferably soybean isoflavones, are present in a content ranging from 0.1% to 10% by weight, the carotenoid, preferably lycopene, is present in a content ranging from 0.02% to 2% by weight, and the vitamin C is present in a content ranging from 0.2% to 12% by weight relative to the total weight of the composition.

For ingestion, numerous embodiments of oral compositions and especially of food supplements are possible. They are formulated via the usual processes for producing coated tablets, gel capsules, gels, emulsions, tablets, capsules, soft capsules, drinkable vials and drinks. In particular, the active agent(s) according to the invention may be incorporated in any other form of food supplements or enriched foods, for example food bars, or compacted or non-compacted powders. The powders may be diluted with water, in soda, dairy products or soybean derivatives, or may be incorporated into food bars.

The active agents according to the invention may be formulated with the usual excipients and components for such oral compositions or food supplements, i.e. especially fatty and/or aqueous components, humectants, thickeners, preserving agents, texture agents, taste agents and/or coating agents, antioxidants, preserving agents and dyes that are common in the food sector.

The formulating agents and excipients for oral compositions, and especially for food supplements, are known in this field and are not the subject of a detailed description herein.

A cosmetic composition that is suitable for the use according to the invention may be in the form of dosage units chosen from a drinkable solution, a syrup, a tablet, a coated tablet, a gel capsule and a capsule.

In a cosmetic composition used according to the invention, the support may be of diverse nature depending on the type of composition under consideration.

In the case of a use of a combination in accordance with the invention, i.e. orally, the use of an ingestible support is preferred.

### Cosmetic process

The present invention describes a cosmetic process for preventing and/or treating hyperpigmentation disorders, comprising a step of orally administering a cosmetic composition comprising a combination (i) of at least one carotenoid, preferably a lycopene, (ii) of at least one phytooestrogen, preferably an isoflavonoid, and (iii) of vitamin C, as an active agent, the said cosmetic composition being suitable for administering a daily dose of from 1 to 25 mg of the said carotenoid, from 10 to 300 mg of the said isoflavonoid and from 10 to 1000 mg of vitamin C.

The described cosmetic process may thus be performed by oral daily administration of the cosmetic composition described above.

The process may comprise a single daily administration of the said cosmetic composition.

According to another embodiment, the administration is repeated, for example 2 to 4 times daily over a day.

The cosmetic process may be applied over a prolonged period of at least 30 successive days, or even 4 to 15 weeks, with, where appropriate, one or more periods of interruption.

The present invention describes a process for preventing and/or treating pigmentation or hyperpigmentation disorders, comprising a step of treatment of a skin surface for aesthetic purposes and a step of oral administration of a cosmetic composition comprising a combination of the invention, especially as defined previously.

For the purposes of the invention, the term "surface skin treatment for aesthetic purposes" means treatments liable to have an aggressive nature with regard to the epidermis and especially liable to cause skin irritation. They may especially be chemical peeling treatments and laser treatments.

An operation of peeling type may consist, for example, in applying to the skin a chemical substance for the purpose of bringing about limited and controlled destruction of the epidermis and of the upper layers of the dermis, in order to improve certain disorders of the cutaneous appearance. As examples of chemical substances that are suitable for peeling, mention may be made of fruit acids: alpha-hydroxy acids (AHAs), and among these glycolic acid, which is suitable in particular for surface peeling, trichloroacetic acid for medium-depth peeling, and phenol for peeling.

A process described in the invention may also comprise, as skin surface treatment step for aesthetic purposes, a peeling step involving the use of ablative and non-ablative lasers. The various lasers that may be used for these treatments are known to those skilled in the art and will not be the subject of a detailed description herein.

A process for preventing and/or treating pigmentation or hyperpigmentation disorders may involve the combined administration of a combination of the invention and of at least a second composition intended to have a beneficial effect on the skin.

This second composition may be administered orally or topically to the skin, and preferably topically. A second composition that is suitable for use in the invention may be any composition comprising at least one active agent, in particular a cosmetic agent, known to have a beneficial effect on the skin. Preferably, a second composition may comprise at least one cosmetic active agent that is capable of exerting a beneficial effect on skin pigmentation, in particular hyperpigmentation or the complexion. Such active agents are known to those skilled in the art.

The invention describes a process comprising the combined, simultaneous, sequential or separate administration orally of a combination of the invention, and orally or topically to the skin, preferably topically, of at least a second composition comprising an active agent, preferably a cosmetic agent, which is suitable for exerting a beneficial effect on the skin.

The examples below are presented as non-limiting illustrations of the field of the invention.

### EXAMPLES

### Examples of oral compositions

### Example 1: formulation of coated tablet type

| **Active material** | **mg/coated tablet** |
|---|---|
| Lycopene (via lactolycopene 2%) | 2 |
| Ascorbic acid | 20 |
| Soybean isoflavones (via Novasoy 400) | 16 |
| | |

| **Excipient for the core of the coated tablet** | |
|---|---|
| Encompress™ | 53 |
| Avicel PH 101 | 53 |
| Aerosil 200 | 4 |
| AcDiSol | 3 |
| Magnesium stearate | 0.5 |
| | |
| **Sugar-coating excipients** (about 85% of the mass of the core) | 242 |

This type of coated tablet may be taken 1 to 3 times a day.

### Example 2: formulation of tablet type

| **Active material** | **mg/tablet** |
|---|---|
| Lycopene (via beadlets 5%) | 3 |
| Ascorbic acid | 30 |
| Soybean isoflavones (via Novasoy 400) | 25 |
| | |

| **Excipient of the tablet core** | |
|---|---|
| Dibasic calcium phosphate dihydrate | 245.80 |
| Microcrystalline Cellulose, silicified 2% | 163.87 |
| Croscarmellose sodium | 21.00 |
| Silicon dioxide | 7.20 |
| Colloidal silicon dioxide | 3.50 |
| Magnesium stearate | 2.80 |
| | |

| **Coating agent** | |
|---|---|
| Opadry white 20A28380 | 21.00 |
| Purified water | qs |
| Opadry Pink 20A240005 | 18 |
| Purified water | qs |

This type of tablet may be taken 1 to 2 times a day.

### Example of demonstration of the activity

### Example 1: Effects of a cosmetic composition comprising a combination of lycopene, a mixture of isoflavonoids and vitamin C on the elimination of cutaneous chloasma

### 1. Materials and methods

### 1.1 Samples

Cosmetic composition in accordance with Example 1.

This composition is suitable for the administration of a combination of lycopene, a mixture of isoflavones extracted from soybean and vitamin C at a daily dose of 6 mg of lycopene, 50 mg of isoflavones and 60 mg of vitamin C.

### 1.2 Subjects

120 healthy female volunteers from 18 to 65 years old, presenting a chloasma, were divided randomly into the "treated" group (n = 60) or into the control group (n = 60).

### 1.3 Test conditions and duration

The subjects of the treated group orally absorbed the cosmetic composition at a rate of two tablets per day for 30 successive days. The subjects do not use during the test any medicament, any functional food, or any cosmetic product in relation with the function of eliminating cutaneous chloasma. The subjects did not modify their eating habits, and ate as usual.

### 1.4 Apparatus and reagent

Measurement of the colour of the chloasma on the face was performed using a colour chart (Y+M+BK; i.e. yellow + magenta + black) as described in *"*Practical standard colour card (1st edition)" published by Surveying and Mappingpress (1992). This colorimetric scale was produced and developed by the Institut des sciences géograpbiques et de recherches des ressources naturelles, académie des sciences de Chine [Institute of geographical sciences and of natural resources research, science academy of China]. This chart was used as standard, according to the following colour determination criteria: degree I (15, 20, 5); degree II (30, 40, 10); degree III (40, 60, 15).

Measurement of the area of the chloasma on the base: the length and breadth of the same chloasma were measured using a graduated ruler in order to calculate the area, expressed in cm².

### 2. Results

### 2.1 General conditions:

On the 120 subjects recruited, 100 subjects were finally analysed, respectively 50 subjects in the "treated" group and 50 subjects in the "control" group.

### 2.2 Measurement of the colour of the chloasma

**Table 1: effect of the cosmetic composition on the colour of the chloasma (+/- SD)**

| Group | Case | Colour before taking the composition (degree) | Colour after taking the composition (degree) | Difference |
|---|---|---|---|---|
| Treated | 0 | 2.06±0.54 | 1.60±0.55**## | 0.46±0.38## |
| Control | 0 | 2.01±0.58 | 1.92±0.55 | 0.09±0.35 |

| | | | | |
|---|---|---|---|---|
| **: P< 0.01: comparison of the results within a group; ##: P< 0.01: comparison of the results at the end of the study between the treated group and the control group. | | | | |

In the light of the results of Table 1, it was shown that the colour of the chloasma decreased very statistically significantly (p<0.01) in the treated group after 30 days of taking the composition.

At the end of the study, the colour of the chloasma, in the treated group, is significantly different (p<0.01) from that in the control group. In other words, the colour of the chloasma in the treated group is statistically less pronounced in the treated group *versus* the control group.

### 2.3 Measurement of the area of the chloasma

**Table 2: Effect of the cosmetic composition on the area of the chloasma (+/- SD)**

| Group | Case | Before taking the composition (cm²) | After taking the composition (cm²) | Difference (cm²) | Percentage reduction (%) |
|---|---|---|---|---|---|
| Treated | 50 | 60.81±49.13 | 50.48±41.87** | 10.33±13.55## | 15.6±16.1## |
| Control | 50 | 59.20±44.53 | 57.26±44.07 | 1.94±7.45 | 2.38±11.4 |

| | | | | | |
|---|---|---|---|---|---|
| **: P< 0.01: comparison of the results before and after the test within the same group; ##: P< 0.01: comparison of the results at the end of the test, between the control group and the treated group. | | | | | |

In the light of the results of Table 2, the area of the chloasma in the treated group was reduced at the end of the test. The reduced area of the chloasma after the test and the percentage reduction, in the treated group, were higher than the corresponding parameters in the control group, with a significant difference (P < 0.01).

### 2.4 Determination of the efficacy

**Table 3: determination of the efficacy of the cosmetic composition on the function of eliminating cutaneous chloasma**

| Group | Case | Efficacy | Inefficacy | Total degree of efficacy |
|---|---|---|---|---|
| Treated | 50 | 32 | 18 | 64.0%## |
| Control | 50 | 7 | 43 | 14.0% |

| | | | | |
|---|---|---|---|---|
| ##: P<0.01: comparison of the treated group and the control group | | | | |

In the light of the results of Table 3, the percentage of volunteers showing an improvement of their chloasma is 64.0% in the treated group as opposed to 14.0% in the control group (p<0.01).

### 3. Conclusion

By comparison with the results before the test, the colour of the chloasma in the treated group was reduced on average by 0.46 ± 0.38 degree; the area of the chloasma was reduced on average by 10.33 ± 13.55 cm², with a percentage reduction of 15.6% ± 16.1%. These improvements are statistically significant. In parallel, the results of the corresponding parameters in the control group were 0.09 ± 0.35 degree, 1.94 ± 7.45 cm², 2.38% ± 11.4%. By comparison with the control group, the colour and the area of the chloasma are statistically less pronounced in the treated group.

The cosmetic composition was effective in the case of 32 individuals in the treated group, with a total degree of efficacy of 64.0%. In parallel, the corresponding parameters in the control group were 7 and 14.0%. A significant difference between the two groups was also shown. In parallel, no new chloasma appeared.

The results as a whole show that the cosmetic composition has a function of eliminating cutaneous chloasma.

## Claims

1. Combination (i) consisting of one carotenoid, preferably lycopene, (ii) one phytooestrogen, preferably an isoflavonoid, and (iii) of vitamin C in a cosmetic composition, for use in the prevention and/or treatment of skin pigmentation disorders, said cosmetic composition being suitable for orally administering a daily dose of from 1 to 25 mg of the said carotenoid, from 10 to 300 mg of the said phytooestrogen and from 10 to 1000 mg of vitamin C, the skin pigmentation disorder being melasma or chloasma.

2. Combination for use according to Claim 1, in the treatment and/or prevention of melasma or chloasma caused by hormonal changes, and more particularly of oestrogens or progestogens or in particular caused by exposure to UV rays.

3. Combination for use according to Claim 2, **characterized in that** the said hormonal changes are caused by the administration of a contraceptive composition.

4. Combination for use according to Claim 1 or 2, **characterized in that** the said hormonal changes are caused by the administration of a hormone replacement composition, for example for a treatment of menopause.

5. Combination for use according to Claims 1 and 2, for treating and/or preventing melasma or chloasma on a photosensitive skin, photoallergic skin or skin subjected to a phototoxicity reaction.

6. Combination for use according to any one of Claims 1 to 5, **characterized in that** the said isoflavonoid is chosen from isoflavones, isoflavanones, rotenoids, pterocarpans, isoflavanes, isoflavan-3-enes, 3-arylcoumarins, 3-aryl-4-hydroxycoumarins, coumestanes, coumaronochromones, α-methyldeoxybenzoins and 2-arylbenzofurans.

7. Combination for use according to any one of Claims 1 to 5, **characterized in that** the isoflavonoid is chosen from genistine, daidzine, genistein, glycitine, equol, formononetine, cuneatine, isoprunetine and prunetine, cajanine, orobol, pratensein, santal, junipegenin A, glycitein, afrormosine, retusine, tectorigenin, irisolidone, jamaicine, in particular chosen from daidzine, daidzein, genistine and genistein, or a mixture thereof.

8. Combination for use according to any one of Claims 1 to 7, **characterized in that** the said at least one isoflavonoid is an isoflavone, or a mixture of isoflavones.

9. Combination for use according to Claim 8, **characterized in that** the said at least one isoflavone is in the form of an extract of soybean isoflavones.

10. Combination for use according to any one of Claims 1 to 9, **characterized in that** the said daily dose of the combination of the said at least one carotenoid, preferably of the said at least one lycopene, of the said at least one phytooestrogen, preferably of the said at least one isoflavonoid, and of vitamin C, is included in a single dosage unit or in a plurality of dosage units of the said cosmetic composition.

11. Combination for use according to any one of Claims 1 to 10, **characterized in that** the said daily dose of the combination of the said at least one carotenoid, preferably of the said at least one lycopene, of the said at least one phytooestrogen, preferably of the said at least one isoflavonoid, and of vitamin C, is included in two dosage units of the said cosmetic composition.

12. Combination for use according to any one of Claims 1 to 11, **characterized in that** the cosmetic composition is in the form of dosage units chosen from a drinkable solution, a syrup, a tablet, a coated tablet, a gel capsule and a capsule.

13. Combination for use according to any one of Claims 1 to 12, **characterized in that** a cosmetic composition is prepared in which the phytooestrogens, preferably the isoflavone compounds, are present in a content ranging from 0.0001% to 50% by weight, preferentially from 0.001% to 30% by weight and preferably from 0.1% to 10% by weight relative to the total weight of the composition.

14. Combination for use according to any one of Claims 1 to 13, **characterized in that** a cosmetic composition is prepared in which the carotenoid, preferably lycopene, is present in a content ranging from 0.0001% to 50% by weight, preferentially from 0.001% to 10% by weight and even more preferably in a content ranging from 0.02% to 2% by weight relative to the total weight of the composition.

15. Combination for use according to any one of Claims 1 to 14, **characterized in that** a cosmetic composition is prepared in which vitamin C is present in a content ranging from 0.0001% to 50% by weight, preferably from 0.1% to 30% by weight and even more preferably in a content ranging from 0.2% to 12% by weight relative to the total weight of the composition.

16. Combination for use according to any one of Claims 1 to 15, **characterized in that** a cosmetic composition is prepared in which the phytooestrogens, preferably soybean isoflavones, are present in a content ranging from 0.1% to 10% by weight, the carotenoids, preferably lycopene, is present in a content ranging from 0.02% to 2% by weight, and the vitamin C is present in a content ranging from 0.2% to 12% by weight relative to the total weight of the composition.

## Patentansprüche

1. Kombination (i) bestehend aus einem Carotinoid, bevorzugt Lycopen, (ii) einem Phytoöstrogen, bevorzugt einem Isoflavonoid, und (iii) Vitamin C in einer kosmetischen Zusammensetzung zur Verwendung in der Prävention und/oder Behandlung von Hautpigmentstörungen, wobei die kosmetische Zusammensetzung zum oralen Verabreichen einer täglichen Dosis von 1 bis 25 mg von dem Carotinoid, von 10 bis 300 mg von dem Phytoöstrogen und von 10 bis 1000 mg Vitamin C geeignet ist, wobei die Hautpigmentstörung Melasma oder Chloasma ist.

2. Kombination zur Verwendung gemäß Anspruch 1 in der Behandlung und/oder Prävention von Melasma oder Chloasma, verursacht durch hormonelle Veränderungen, und stärker besonders von Östrogenen oder Progestogenen, oder insbesondere verursacht durch Einwirkung von UV-Strahlen.

3. Kombination zur Verwendung gemäß Anspruch 2, **dadurch gekennzeichnet, dass** die hormonellen Veränderungen durch die Verabreichung einer kontrazeptiven Zusammensetzung verursacht werden.

4. Kombination zur Verwendung gemäß Anspruch 1 oder 2, **dadurch gekennzeichnet, dass** die hormonellen Veränderungen durch die Verabreichung einer Hormonersatzzusammensetzung, zum Beispiel für eine Behandlung von Menopause, verursacht werden.

5. Kombination zur Verwendung gemäß den Ansprüchen 1 und 2 zur Behandlung und/oder Prävention von Melasma oder Chloasma auf einer fotoempfindlichen Haut, fotoallergischen Haut oder Haut, welche einer Fototoxizitätsreaktion ausgesetzt ist.

6. Kombination zur Verwendung gemäß einem der Ansprüche 1 bis 5, **dadurch gekennzeichnet, dass** das Isoflavonoid aus Isoflavonen, Isoflavanonen, Rotenoiden, Pterocarpanen, Isoflavanen, Isoflavan-3-enen, 3-Arylcoumarinen, 3-Aryl-4-hydroxycoumarinen, Coumestanen, Coumaronochromonen, α-Methyldeoxybenzoinen und 2-Arylbenzofuranen ausgewählt ist.

7. Kombination zur Verwendung gemäß einem der Ansprüche 1 bis 5, **dadurch gekennzeichnet, dass** das Isoflavonoid ausgewählt ist aus Genistin, Daidzin, Genistein, Glycitin, Equol, Formononetin, Cuneatin, Isoprunetin und Prunetin, Cajanin, Orobol, Pratensein, Santal, Junipegenin A, Glycitein, Afrormosin, Retusin, Tectorigenin, Irisolidon, Jamaicin, insbesondere ausgewählt ist aus Daidzin, Daidzein, Genistin und Genistein, oder einem Gemisch davon.

8. Kombination zur Verwendung gemäß einem der Ansprüche 1 bis 7, **dadurch gekennzeichnet, dass** das mindestens eine Isoflavonoid ein Isoflavon oder ein Gemisch von Isoflavonen ist.

9. Kombination zur Verwendung gemäß Anspruch 8, **dadurch gekennzeichnet, dass** das mindestens eine Isoflavon in der Form eines Extrakts von Sojabohnen-Isoflavonen ist.

10. Kombination zur Verwendung gemäß einem der Ansprüche 1 bis 9, **dadurch gekennzeichnet, dass** die tägliche Dosis der Kombination von dem mindestens einen Carotinoid, bevorzugt von dem mindestens einen Lycopen, von dem mindestens einen Phytoöstrogen, bevorzugt von dem mindestens einen Isoflavonoid, und von Vitamin C in einer einzelnen Dosierungseinheit oder in einer Vielzahl von Dosierungseinheiten der kosmetischen Zusammensetzung eingeschlossen ist.

11. Kombination zur Verwendung gemäß einem der Ansprüche 1 bis 10, **dadurch gekennzeichnet, dass** die tägliche Dosis der Kombination von dem mindestens einen Carotinoid, bevorzugt von dem mindestens einen Lycopen, von dem mindestens einen Phytoöstrogen, bevorzugt von dem mindestens einen Isoflavonoid, und von Vitamin C in zwei Dosierungseinheiten der kosmetischen Zusammensetzung eingeschlossen ist.

12. Kombination zur Verwendung gemäß einem der Ansprüche 1 bis 11, **dadurch gekennzeichnet, dass** die kosmetische Zusammensetzung in der Form von Dosierungseinheiten, ausgewählt aus einer trinkbaren Lösung, einem Sirup, einer Tablette, einer überzogenen Tablette, einer Gelkapsel und einer Kapsel, ist.

13. Kombination zur Verwendung gemäß einem der Ansprüche 1 bis 12, **dadurch gekennzeichnet, dass** eine kosmetische Zusammensetzung hergestellt wird, in welcher die Phytoöstrogene, bevorzugt die Isoflavon-Verbindungen, in einem Gehalt in einem Bereich von 0,0001 Gew.-% bis 50 Gew.-%, vorzugsweise von 0,001 Gew.-% bis 30 Gew.-% und bevorzugt von 0,1 Gew.-% bis 10 Gew.-%, relativ zum Gesamtgewicht der Zusammensetzung, vorhanden sind.

14. Kombination zur Verwendung gemäß einem der Ansprüche 1 bis 13, **dadurch gekennzeichnet, dass** eine kosmetische Zusammensetzung hergestellt wird, in welcher das Carotinoid, bevorzugt Lycopen, in einem Gehalt in einem Bereich von 0,0001 Gew.-% bis 50 Gew.-%, vorzugsweise von 0,001 Gew.-% bis 10 Gew.-% und noch stärker bevorzugt in einem Gehalt in einem Bereich von 0,02 Gew.-% bis 2 Gew.-%, relativ zum Gesamtgewicht der Zusammensetzung, vorhanden ist.

15. Kombination zur Verwendung gemäß einem der Ansprüche 1 bis 14, **dadurch gekennzeichnet, dass** eine kosmetische Zusammensetzung hergestellt wird, in welcher Vitamin C in einem Gehalt in einem Bereich von 0,0001 Gew.-% bis 50 Gew.-%, bevorzugt von 0,1 Gew.-% bis 30 Gew.-% und noch stärker bevorzugt in einem Gehalt in einem Bereich von 0,2 Gew.-% bis 12 Gew.-%, relativ zum Gesamtgewicht der Zusammensetzung, vorhanden ist.

16. Kombination zur Verwendung gemäß einem der Ansprüche 1 bis 15, **dadurch gekennzeichnet, dass** eine kosmetische Zusammensetzung hergestellt wird, in welcher die Phytoöstrogene, bevorzugt Sojabohnen-Isoflavone, in einem Gehalt in einem Bereich von 0,1 Gew.-% bis 10 Gew.-% vorhanden sind, die Carotinoide, bevorzugt Lycopen, in einem Gehalt in einem Bereich von 0,02 Gew.-% bis 2 Gew.-% vorhanden sind, und das Vitamin C in einem Gehalt in einem Bereich von 0,2 Gew.-% bis 12 Gew.-% vorhanden ist, relativ zum Gesamtgewicht der Zusammensetzung.

## Revendications

1. Combinaison consistant en (i) au moins un caroténoïde, de préférence un lycopène, (ii) au moins un phytoestrogène, de préférence un isoflavonoïde et (iii) vitamine C dans une composition cosmétique, pour une utilisation dans la prévention et/ou le traitement des désordres pigmentaires cutanés, ladite composition cosmétique étant adaptée à l'administration par voie orale d'une dose quotidienne de 1 à 25 mg dudit caroténoïde, de 10 à 300 mg dudit phytoestrogène et de 10 à 1000 mg de vitamine C, et le désordre pigmentaire cutané étant un mélasma ou un chloasma.

2. Combinaison pour une utilisation selon la revendication 1, dans le traitement et/ou la prévention d'un mélasma ou d'un chloasma provoqué par des changements hormonaux, plus particulièrement en oestrogènes ou progestatifs, ou en particulier provoqués par une exposition aux rayons UV.

3. Combinaison pour une utilisation selon la revendication 2, **caractérisée en ce que** lesdits changements hormonaux sont provoqués par l'administration d'une composition contraceptive.

4. Combinaison pour une utilisation selon la revendication 1 ou 2, **caractérisée en ce que** lesdits changements hormonaux sont provoqués par l'administration d'une composition de substitution hormonale, par exemple pour un traitement de la ménopause.

5. Combinaison pour une utilisation selon les revendications 1 et 2, pour traiter et/ou prévenir un mélasma ou un chloasma sur une peau photosensible, une peau photo-allergique ou une peau sujette à une réaction de phototoxicité.

6. Combinaison pour une utilisation selon l'une quelconque des revendications 1 à 5, **caractérisée en ce que** ledit isoflavonoïde est choisi parmi les isoflavones, les isoflavanones, les roténoïdes, les ptérocarpans, les isoflavanes, les isoflavan-3-ènes, les 3-arylcoumarines, les 3-aryl-4-hydroxycoumarines, les coumestanes, les coumaronochromones, les α-méthyldésoxybenzoïnes et les 2-arylbenzofuranes.

7. Combinaison pour une utilisation selon l'une quelconque des revendications 1 à 5, **caractérisée en ce que** l'isoflavonoïde est choisi parmi la génistine, la daidzine, la génistéine, la glycitine, l'équol, la formononétine, la cunéatine, l'isoprunétine et la prunétine, la cajanine, l'orobol, la pratenséine, le santal, la junipégénine A, la glycitéine, l'afrormosine, la rétusine, la tectorigénine, l'irisolidone, la jamaïcine, en particulier choisi parmi la daidzine, la daidzéine, la génistine et la génistéine, ou un de leurs mélanges.

8. Combinaison pour une utilisation selon l'une quelconque des revendications 1 à 7, **caractérisée en ce que** ledit au moins un isoflavonoïde est une isoflavone, ou un mélange d'isoflavones.

9. Combinaison pour une utilisation selon la revendication 8, **caractérisée en ce que** ladite au moins une isoflavone est sous la forme d'un extrait d'isoflavones de soja.

10. Combinaison pour une utilisation selon l'une quelconque des revendications 1 à 9, **caractérisée en ce que** ladite dose quotidienne de la combinaison dudit au moins un caroténoïde, de préférence dudit au moins un lycopène, dudit au moins un phytoestrogène, de préférence dudit au moins un isoflavonoïde, et de la vitamine C est comprise dans une seule unité de dosage ou dans une pluralité d'unités de dosage de ladite composition cosmétique.

11. Combinaison pour une utilisation selon l'une quelconque des revendications 1 à 10, **caractérisée en ce que** ladite dose quotidienne de la combinaison dudit au moins un caroténoïde, de préférence dudit au moins un lycopène, dudit au moins un phytoestrogène, de préférence dudit au moins un isoflavonoïde, et de la vitamine C est comprise dans deux unités de dosage de ladite composition cosmétique.

12. Combinaison pour une utilisation selon l'une quelconque des revendications 1 à 11, **caractérisée en ce que** la composition cosmétique se présente sous la forme d'unités de dosage choisies parmi une solution buvable, un sirop, un comprimé, une dragée, une gélule et une capsule.

13. Combinaison pour une utilisation selon l'une quelconque des revendications 1 à 12, **caractérisée en ce qu'**il est préparé une composition cosmétique dans laquelle les phytoestrogènes, de préférence les composés isoflavones, sont présents dans une teneur allant de 0,0001 à 50 % en poids, préférentiellement de 0,001 à 30 % en poids et de préférence de 0,1 à 10 % en poids par rapport au poids total de la composition.

14. Combinaison pour une utilisation selon l'une quelconque des revendications 1 à 13, **caractérisée en ce qu'**il est préparé une composition cosmétique dans laquelle le caroténoïde, de préférence le lycopène, est présent dans une teneur allant de 0,0001 à 50 % en poids, préférentiellement de 0,001 à 10 % en poids, de façon encore plus préférée en une teneur allant de 0,02 % à 2 % en poids par rapport au poids total de la composition.

15. Combinaison pour une utilisation selon l'une quelconque des revendications 1 à 14, **caractérisée en ce qu'**il est préparé une composition cosmétique dans laquelle la vitamine C est présente dans une teneur allant de 0,0001 à 50 % en poids, de préférence de 0,1 à 30 % en poids et de façon encore plus préférée en une teneur allant de 0,2 à 12 % en poids par rapport au poids total de la composition.

16. Combinaison pour une utilisation selon l'une quelconque des revendications 1 à 15, **caractérisée en ce qu'**il est préparé une composition cosmétique dans laquelle les phytoestrogènes, de préférence les isoflavones de soja, sont présents dans une teneur allant de 0,1 à 10 % en poids, le caroténoïde, de préférence le lycopène, est présent dans une teneur allant de 0,02 % à 2 % en poids, et la vitamine C est présente dans une teneur allant de 0,2 à 12 % en poids par rapport au poids total de la composition.
